(19) Europäisches Patentamt
European Patent Office
Office européen des brevets

(11) EP 0 578 148 B1

# (12) EUROPÄISCHE PATENTSCHRIFT

(45) Veröffentlichungstag und Bekanntmachung des Hinweises auf die Patenterteilung:
**19.04.2000 Patentblatt 2000/16**

(51) Int. Cl.$^7$: **G01N 33/543**, G01N 33/547, G01N 33/531

(21) Anmeldenummer: **93110595.1**

(22) Anmeldetag: **02.07.1993**

(54) **Biologisch erkennende Schichten auf Festphasen sowie ein Verfahren zur Herstellung dieser Schichten**

Biologically recognising layers on solid phases and method for producing same

Couches qui reconnaissent biologiquement sûr les phases solides et procédé pour la production

(84) Benannte Vertragsstaaten:
**BE CH DE DK ES FR GB GR IE IT LI LU NL PT**

(30) Priorität: **10.07.1992 CH 217892**

(43) Veröffentlichungstag der Anmeldung:
**12.01.1994 Patentblatt 1994/02**

(73) Patentinhaber:
**F. HOFFMANN-LA ROCHE AG**
**4002 Basel (CH)**

(72) Erfinder:
- **Barner, Richard**
  **CH-4108 Witterswil (CH)**
- **Huber, Walter**
  **CH-4303 Kaiseraugst (CH)**
- **Hübscher, Josef**
  **CH-4208 Nunningen (CH)**
- **Hurst, Jürg**
  **CH-4057 Basel (CH)**
- **Schlatter, Daniel**
  **CH-4104 Oberwil (CH)**

(74) Vertreter:
**Jung, Michael, Dr. et al**
**Roche Diagnostics GmbH**
**Patentabteilung**
**Sandhofer Strasse 116**
**68305 Mannheim (DE)**

(56) Entgegenhaltungen:
**EP-A- 0 408 078**
**EP-A- 0 416 730**
**EP-A- 0 485 874**
**WO-A-91/13358**
**FR-A- 2 567 133**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die vorliegende Erfindung betrifft biologisch erkennende Schichten auf Festphasen, sowie ein Verfahren zur Herstellung dieser Schichten.

Festphasen mit biologisch erkennenden Schichten werden in bioanalytischen Verfahren eingesetzt, welche sich unter dem Sammelbegriff Assay-Technologie zusammenfassen lassen. Beispiele hierfür sind: Enzym-Immunoassays, Fluoreszenz-Immunoassays, Radio-Immunoassays, je nach Markierungsart der beteiligten Moleküle.

In der neueren bioanalytischen Literatur werden in zunehmendem Masse für derartige Nachweise sogenannte Affinitäts-Sensoren beschrieben, welche im Gegensatz zur bestehenden Assay-Technologie einen direkten Nachweis ohne Verwendung einer Markierung, und ohne Wasch- und Separationsschritte erlauben. Derartige Affinitäts-Sensoren bestehen im allgemeinen aus einem physikalischen Wandler, speziell z.B. aus einem piezoelektrischen Wandler [Anal.Chem. 63 (1991) 393 A], aus einem optischen Wandler [Sens. Actuators 4 (1983) 299; Thin Solid Films 126 (1985) 205; Biosensors and Biosensing 6 (1991) 215] oder aus einem elektrochemischen Wandler [ Biosensors & Bioelectronics 6 (1991) 55]), welcher eng verknüpft ist mit der oben erwähnten biologisch erkennenden Schicht.

Da das Sensorsignal dieser Affinitäts-Sensoren direkt proportional ist einer Aenderung der Oberflächenbelegung durch Adsorption bzw. Desorption von Molekülen, sind an die biologisch erkennenden Schichten in der Sensorik besonders hohe Ansprüche zu stellen. Während in der Assay-Technologie die biologisch erkennende Schicht vielfach durch physikalische Adsorption der biologisch erkennenden Moleküle oder Molekülfragmente an die Festphase gebunden wird, wird für die Sensorik zwingend gefordert, dass diese erkennenden Moleküle kovalent an die Oberfläche gebunden werden, den, um eine Desorption der erkennenden Moleküle von der Oberfläche zu verhindern. Bei diesem kovalenten Immobilisieren eines biologisch erkennenden Moleküls auf der Oberfläche müssen folgende Gesichtspunkte berücksichtigt werden:

Die Empfindlichkeit der Detektion und der dynamische Bereich dieser Affinitäts-Sensoren hängen neben dem Auflösungsvermögen des Wandlers bezüglich einer Aenderung in der Oberflächenbelegung auch von der Affinität der erkennenden Schicht für den nachzuweisenden Analyten und von der Bindungskapazität dieser Oberfläche für ein Binden des Analyten ab. Unter Bindungskapazität wird die Anzahl aktiver Bindungsstellen pro Flächeneinheit verstanden.

In der Literatur sind verschiedene Verfahren beschrieben, nach denen biologisch erkennende Moleküle kovalent auf der Oberfläche immobilisiert werden. Diese Verfahren bedingen im allgemeinen eine der Immobilisierung vorangehende biologische oder chemische Modifikation der Moleküle.

Tische Beispiele für das kovalente Immobilisieren von Antikörpern oder Antikörperfragmenten an einem Immunosensor sind:

i) Oxidation der Zuckerreste am Fc-Teil und Immobilisierung der Antikörper via die entstehenden Aldehydfunktionen auf eine Oberfläche, welche Hydrazingruppen als komplementäre reaktive Gruppen trägt.

ii) Umsetzung derart erzeugter Aldehydfunktionen mit geeigneten Biotinderivaten. Aufgrund der bekannt hohen Affinität des Biotins zu Avidin und Streptavidin haften derart modifizierte Antikörper extrem gut auf einer Avidin/Streptavidinoberfläche.

iii) Behandlung der Antikörper mit Pepsin, Reduktion der dabei entstehenden Fab2 - Fragmente zu Fab' - Fragmenten und Verankerung dieser Fab'-Fragmente über deren freie SH-Gruppen auf eine entsprechend mit funktionellen Gruppen (z.B.Dithiopyridylgruppen) ausgestattete Oberfläche.

WO91/13358 beschreibt ein Verfahren zur Immobilisierung einer biologisch erkennenden Schicht auf einer Festphase, wobei primäre Moleküle, die eine Bindungsstelle aufweisen, wie beispielsweise Antikörper, an die Oberfläche dieser Festphase gebunden werden und dann um diese primären Moleküle herum ein dichtes Netz von sekundären Molekülen, so daß die Bindungsstelle der primären Moleküle von der Oberfläche der Festphase wegweist. Um einer sterischen Behinderung der Bindungsstelle von Antikörpern zuvorzukommen, ist es notwendig ein Oligosaccharid durch Einführung eines Spacers chemisch zu modifizieren.

Die bekannten Verfahren haben den Nachteil, dass mit der chemischen und/oder biochemischen Modifikation der biologisch erkennenden Moleküle ein wesentlicher Aktivitätsverlust einhergeht.

Der vorliegenden Erfindung liegt daher die Aufgabe zugrunde, eine gerichtete, kovalent immobilisierte Schicht von biologisch erkennenden, unmodifizierten Molekülen mit hoher Bindungskapazität für das zu erkennende Molekül auf einer Festphase bereitzustellen. Eine hohe Affinität und Bindungskapazität setzt eine hohe Dichte an aktiven, spezifischen Bindungsstellen voraus.

Ueberraschenderweise wurde nun gefunden, dass eine solche gerichtete, kovalent immobilisierte Schicht von biologisch erkennenden Molekülen auch verwirklicht werden kann, ohne dass durch chemische und / oder biochemische Modifikation funktionelle Gruppen für das kovalente Binden am erkennenden Molekül geschaffen werden.

Gegenstand der Erfindung sind biologisch erkennende Schichten, die auf einer Festphase kovalent immobilisiert sind, wobei die Oberfläche der Festphase mit einer organischen Zusatzschicht versehen ist, die die für eine Proteinimmobilisierung geeigneten funktionellen Gruppen in hoher Dichte besitzt, und die aus biologisch erkennenden Molekülen bestehen, die den Analyten erkennende und bindende Molekülbereiche und den Analyten nicht erkennende und bindende Molekülbereiche aufweisen, wobei die biologisch erkennenden Moleküle in einer Vorzugsrichtung derart kovalent immobilisiert sind,

(a) dass die den Analyten erkennenden und bindenden Molekülbereiche von der Oberfläche der Festphase abgewandt sind und nicht durch die kovalente Bindung verändert sind,

(b) dass die den Analyten nicht erkennenden Molekülbereiche an eine die erkennenden Moleküle ausrichtende Schicht gebunden sind, welche spezielle Bindungstellen für diese gerichtete Adsorption der biologisch erkennenden Moleküle aufweist und aus ausrichtenden Molekülen besteht, die an der organischen Zusatzschicht immobilisiert sind, und

(c ) dass die biologisch erkennenden Moleküle untereinander und mit den ausrichtenden Molekülen über chemisch modifizierte Trägermoleküle quervernetzt sind, wobei die Quervernetzung unter Verwendung von Trägermolekülen photochemisch erfolgt ist, welche Trägermoleküle mehrere photoaktivierbare Gruppen pro Molekül tragen und eine hohe Wasserlöslichkeit, insbesondere im photoaktivierbaren Teil des Moleküls, aufweisen.

Erfindungsgemäss wird das gerichtete, kovalente Immobilisieren von biologisch erkennenden Molekülen dadurch erreicht, dass das zu immobilisierende Molekül nach geeigneter Modifikation der Festphasenoberfläche über die, den Analyten nicht erkennenden Molekülbereiche adsorbiert wird. Nach diesem Adsorptionsschritt erfolgt der kovalente Bindungsschritt über die den Analyten nicht erkennende Bereiche unter Zuhilfenahme spezifischer, diesem Immobilisierungsverfahren besonders angepasster sogenannter Crosslinking - Reagenzien, die über Trägermoleküle in das Verfahren eingeführt werden. Die Trägermoleküle können vor, gleichzeitig mit oder nach der gerichteten Adsorption der Analyt erkennenden Moleküle auf der Oberfläche der Festphase adsorbiert oder gebunden werden.

Die vorliegende Erfindung beschreibt somit eine Schicht von erkennenden Molekülen, sowie ein Verfahren zur Herstellung dieser Schicht, nach dem unmodifizierte, erkennende Biomoleküle (z.B. Antikörper, Rezeptoren, DNA-Moleküle) gerichtet auf der Oberfläche immobilisiert werden.

Das Verfahren ist grundsätzlich anwendbar auf alle erkennenden Moleküle, die einen ersten Analyt erkennenden Molekülbereich und einen von dem ersten räumlich gut separierten zweiten Molekülbereich besitzen, über den sich das erkennende Molekül auf eine geeignet modifizierte Oberfläche adsorbieren lässt. Es lässt sich zeigen, dass diese strukturellen Voraussetzungen nahezu in allen erkennenden Biomolekülen vorhanden sind.

Ligand-erkennende Rezeptoren der Zellmembran verfügen z.B. über einen hydrophilen Molekülteil, welcher den Ligand-erkennenden Bereich beinhaltet und über einen meist hydrophoben Transmembranteil. Eine physikalische Adsorption derartiger Rezeptoren auf hydrophobe Oberflächen erfolgt bevorzugt über diesen Transmembranteil.

Gegenstand der Erfindung ist außerdem ein Verfahren zur Herstellung biologisch erkennender Schichten , weiches dadurch gekennzeichnet ist, dass vor der Quervernetzung die gerichtete Adsorption der biologisch erkennenden Moleküle erfolgt, die dadurch erreicht wird, daß vor der Adsorption der biologisch erkennenden Moleküle auf der Oberfläche der Festphase durch chemische bzw. biochemische Behandlung Bindungsstellen geschaffen werden, welche den Analyten nicht erkennende Molekülbereiche der biologisch erkennenden Moleküle adsorptiv binden, wobei die Quervernetzung unter Verwendung von Trägermolekülen photochemisch erfolgt, welche Trägermoleküle mehrere photoaktivierbare Gruppen pro Molekül tragen und eine hohe Wasserlöslichkeit, insbesondere im photoaktivierbaren Teil des Moleküls, aufweisen.

Antikörper, verfügen über Fab-Teile mit den Antigen/Hapten erkennenden Bereichen und über den für die Antigen/Hapten-Erkennung unwichtigen Fc-Teil. Durch Immobilisierung von Fc-Teil-erkennenden Molekülen kann die Oberfläche der Festphase so gestaltet werden, dass Antikörper über den Fc-Teil gerichtet auf die Oberfläche adsorbiert werden können.

Bei DNA-Molekülen wird nur eine bestimmte Sequenz für die Erkennung des komplementären Stranges verwendet. Sequenzen (z.B. 3-10 Basen), welche auf einem derartigen Strang nicht für die Erkennung des komplementären Stranges verwendet werden, können für die gerichtete Adsorption verwendet werden, wenn vor der Adsorption das entsprechende kurze komplementäre Teilstück (z.B. 3-10 Basen) auf der Oberfläche immobilisiert wird.

Schliesslich werden erkennende Biomoleküle in neuerer Zeit immer häufiger biotechnologisch hergestellt. Durch gentechnologische Verfahren kann für die biotechnologische Produktion des Erkennungsmoleküls der verwendete Mikroorganismus so verändert werden, dass an spezifischen Stellen dieses Erkennungsmoleküls zusätzliche, spezifisch für die Adsorption an eine Oberfläche geeignete Bereiche synthetisiert werden.

Am Beispiel einer Antigen/Hapten erkennenden Schicht, bei der Antikörper kovalent auf der Oberfläche einer Festphase immobilisiert sind, wird die Erfindung näher erläutert. Als Festphase eignet sich die Oberfläche eines physikalischen Wandlers.

Die gerichtete Adsorption von Antikörpern wird dadurch erreicht, dass die Oberfläche der Festphase zuerst mit einer Monolage von Proteinen versehen wird, welche eine spezifische Affinität zum Fc-Teil von Antikörpern aufweisen. Typische Vertreter dieser Klasse von Proteinen, die auf Grund ihrer Affinität zu Fc-Teilen von Antikörpern in der Lage sind Antikörper an einer Oberfläche auszurichten, sind z.B. Protein A, Protein G, Fc-Teil-erkennende Antikörper bzw. Antikörperfragmente oder Rezeptoren. Mit Vorteil wird dabei mit solchen Proteinen innerhalb dieser Klasse gearbeitet, welche einerseits mehrere Bindungsstellen für den Fc-Teil von Antikörpern besitzen (z.B. Protein A mit vier Bindungsstellen pro Molekül) und andererseits einen wesentlich geringeren Raumbedarf (Flächenbedarf) aufweisen als die nachfolgend zu immobilisierenden Antikörper (z.B. Protein A mit einer globulären Struktur mit einem Durchmesser von ca. 5 nm im Vergleich zu Antikörpern vom IgG-Typ mit einer Separation der Antigen erkennenden Bereiche von ca. 17 nm).

Sind diese Bedingungen optimal erfüllt, wird auch bei einem nicht richtungskontrollierten kovalenten Immobilisieren dieses Proteins auf der Oberfläche eine genügend hohe Dichte an Fc-Teil-spezifischen Bindungsstellen auf der Oberfläche erreicht.

Verfahren für ein derartiges, bezüglich der Ausrichtung auf der Oberfläche nicht kontrolliertes Immobilisieren Fc-Teil-erkennender Proteine sind hinlänglich bekannt. So wird im allgemeinen die Oberfläche der Festphase mit einer dünnen organischen Zusatzschicht versehen, welche die für eine Proteinimmobilisierung geeigneten funktionellen Gruppen (z.B. -COOH oder $-NH_2$) in hoher Dichte besitzt. Das Aufbringen einer derartigen Zusatzschicht kann z.B. nach den aus der Chromatographie bekannten Silanisierungsverfahren und nach den in der neueren Literatur beschriebenen Self-Assembling-Verfahren aus Lösung oder nach den mit den Sammelbegriffen Chemical Vapor Deposition, Plasma Induced Chemical Vapor Deposition, Plasma Induced Polymerization charakterisierbaren Verfahren aus der Gasphase erfolgen . Das kovalente Immobilisieren der Fc-Teil-erkennenden Proteine erfolgt nach bekannten Verfahren aus der Affinitätschromatografie, indem die funktionellen Gruppen an der Oberfläche geeignet aktiviert und anschliessend mit funktionellen Gruppen (z.B. $-NH_2$ oder -COOH) am Protein zur Reaktion gebracht werden.

Auf derartigen Oberflächen können nun Antikörper gerichtet verankert werden, wobei das Verankern auf der Affinität des immobilisierten Proteins zum Fc-Teil der Antikörper beruht. Es ist jedoch hinlänglich bekannt, dass die Affinitätskonstanten für derartige Komplexe (z.B. IgG-Protein A) im allgemeinen nicht ausreichen um eine Desorption des IgG-Moleküls zu unterbinden. Hinzu kommt, dass verschiedene Antikörper-Subklassen nur unter ganz speziellen Pufferbedingungen via ihren Fc-Teil an diese Proteine gebunden werden (z.B. hoher pH-Wert, hohe Salzkonzentrationen), wobei diese Antikörper bei einem nachfolgenden Pufferwechsel wieder (beispielsweise unter physiologischen Bedingungen) rasch desorbieren.

Wie vorangehend erläutert, hat sich dieser gerichteten Adsorption ein zweiter Schritt, die kovalente Immobilisierung der ausgerichteten Moleküle anzuschliessen. Es ist Klar, dass dieser Vernüpfungsvorgang so zu erfolgen hat, dass dadurch die Antigen-bindenden Bereiche und somit die Aktivität der Antikörper nicht beeinflusst werden. Herkömmliche chemische Verfahren für ein derartiges Crosslinking haben im allgemeinen den Nachteil einer hohen Unspezifität, das heisst das Crosslinking erfolgt auch in den Antigen/Hapten-bindenden Bereichen und vermindert dadurch weitgehend die Aktivität der Antikörper.

Die Durchführung des Verknüpfungsschritts erfolgt durch eine selektive Crosslinking-Technologie bestehend aus:

i) der Bereitstellung von bifunktionellen Reagenzien, die sowohl eine photolytisch aktivierbare Gruppe als auch eine chemisch reaktive Gruppe besitzen und eine hohe Wasserlöslichkeit besonders des photolytisch aktivierbaren Molekülteils aufweisen.

ii) der gleichzeitigen Bereitstellung eines Verfahrens, durch welches dieses photolytisch induzierte Crosslinking gezielt an den den Analyten nicht erkennenden Bereichen erfolgt.

Das Crosslinking Verfahren, welches zu Oberflächen führt auf denen Antikörper kovalent und gerichtet immobilisiert sind, basiert auf der Verwendung von Verbindungen mit der allgemeinen Formel:

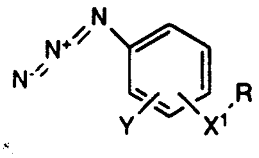

I

worin $X^1$ eine Carbonyl- ( >C=O) oder Sulfonylgruppe ( $>SO_2$) und Y=H,Y' oder $X^1$-Y' bedeutet. Y' ist eine Hydroxy- bzw. Alkoxygruppe (-O-Y") oder eine Aminogruppe ( - NH-Y"), dabei bedeutet Y"=H oder eine wasserlöslichmachende Gruppe vom Typ $(CH_2)_nA$. n=1-6

Dabei ist A ein Glykol- bzw. Oligoethylenglykol-Substituent, eine tert. bzw. quaternäre Aminogruppe wie Pyridyl, Dialkylamino bzw. N-Alkylpyridinium, Trialkylammonium. Alkyl bedeutet einen niederen Alkylrest ca. $C_1$ - $C_4$.

Die Gruppe R ist eine funktionelle Gruppe der allgemeinen Formel:

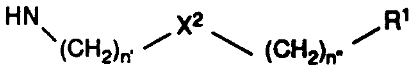

II

worin $X^2$ eine Disulfid- (-S-S-) oder Methylengruppe ($-CH_2-$) bedeuten.

$R^1$ bedeutet ein Amino- ($-NH-R^2$) oder Carboxylderivat ($-CO-R^3$). Dabei bedeutet $R^2$=H oder eine derivatisierte Carboxyalkanoylgruppe ($-CO-(CH_2)_nCO-R^3$).

$CO-R^3$ ist eine aktivierte Carboxylgruppe wie z.B. ein Säurehalogenid, Imidazolid, Hydrazid, Anhydrid, eine mit einer Dithiopyridylgruppe ($-NH-(CH_2)n'''$-S-S-Pyridyl) derivatisierte Carboxylgruppe oder ein reaktiver Ester mit z.B. Hydroxysuccinimid, Isoharnstoff, Hydroxysuccinimidsulfonsäure.

n',n",n'''=1-6.

Wenn $X^2$ eine Methylengruppe bedeutet, kann $R^3$ eine Disulfidgruppe enthalten, wobei $R^3$ z.B. ein Cystaminderivat $-NH-(CH_2)_2-S-S-(CH_2)_2-NH-R^2$ bedeutet, worin $R^2$ die obenstehende Bedeutung hat und das in $R^2$ enthaltene $R^3$ keine Disulfidgruppe enthält.

Für Y=H ist $X^1$-R ( ohne $R^3$ ) hydrophil ( z.B. $X^1=-SO_2-$ oder $R^1$=tert. Amin oder quartäres Ammonium).

Für hydrophiles $X^1$-R ( ohne $R^3$ ) kann Y gewünschtenfalls auch $X^1$-R sein. (doppelte Verankerung)

Die Aminogruppe $R^1$ kann auch in andere reaktive Gruppen umgewandelt werden wie z.B. in ein Isocyanat, Isothiocyanat, Vinylsulfonsäureamid ($-NH-SO_2-CH=CH_2$), Maleinimid, halogensubstituierte Triazinamino-, Pyrimidinamino- oder Pyridinaminoverbindungen (z.B. Dichlortriazin), 2-Halogencarbonsäurederivate (z.B. mit 2-Halogenessigsäurehalogenid, 2-Halogenpropionsäurehalogenid und dergleichen), Monoamide aus Dicarbonsäuredihalogeniden, Epoxide z.B. mit Epichlorhydrin oder ein Cyclohexendionderivat via Michaeladdition an ein Chinon.

| | | |
|---|---|---|
| $X^1$ = -CO- | | |
| $X^1 = -SO_2-$ | | |
| $X^2$ = -S-S- | | |
| $X^2 = -(CH_2)-$ | | |
| Y = -H | | $A = -O-(CH_2)_2-O-H$ |
| Y = -Y' | | $A = -O-[(CH_2)_2-O]_n-H$ |
| Y= -CO-Y' = $-X^1$-Y' | Y' = -O-Y" Y" = -H | A = -N(Alkyl)2 |
| $Y = -SO_2-Y' = -X^1-Y'$ | $Y' = -NH-Y"Y" = -(CH_2)_n-A$ | $A = -N^+(Alkyl)_3$ |
| | | A=-Pyridin |
| | | A = -Pyridinium(N-Alkyl) |
| $R^1 = -CO-R^3$ | $R^2$ = H | $COR^3$ = CO-Cl |

(fortgesetzt)

| | | |
|---|---|---|
| $R^1$ = -NH-$R^2$ | $R^2$ = -CO-$(CH_2)_n$-CO-$R^3$ | $COR^3$ = CO-O-Acyl<br>$COR^3$ = CO-Isoharnstoff<br>$COR^3$=CO -OSu<br>$COR^3$ = CO -OSu(SO3H)<br>$COR^3$ = CO -NH-$NH_2$<br>$COR^3$ = CO -NH-$(CH_2)_{n'''}$-S-S-Pyridyl<br>$COR^3$ = CO-NHCON$H_2$<br>$COR^3$ = CO-Imidazolyl |
| $X^2$ = -$CH_2$-, | | |

$R^1$ = $COR^3$ = CO-NH-$(CH_2)_2$-S-S-$(CH_2)_2$-NH-$R^2$ ($R^2$ = H, CO-$(CH_2)_n$-CO-$R^3$) wobei $R^3$ wie in Spalte 3 definiert ist mit Ausnahme der Disulfidverb.)
$R^1$ = Pyridinium(N-$CH_2$-CO-$R^3$)

| | |
|---|---|
| $R^1$ = | -N=C=O |
| | -N=C=S |
| ⇒ | $CH_2$=CH-$SO_2$-NH- |
| | -Maleinimidyl |
| | -NH-CO-CH(Cl)-Alkyl |
| | -NH-CO-Alkylen-CO-Cl |
| | -NH-$CH_2$-Oxiran |
| | -NH-Cyclohexendion |
| | -NH-Dichlortriacinyl |

Das hier beanspruchte Verfahren zur gerichteten Immobilisierung unter Verwendung der oben spezifizierten heterobifunktionellen Reagenzien geht aus von Oberflächen, auf denen Fc-Teil - erkennende Proteine kovalent immobilisiert sind. Das erfindungsgemässe Verfahren zeichnet sich dadurch aus, dass für das photolytisch induzierte Crosslinking weder diese Proteinschicht noch die nachfolgend über den Fc-Teil an diese Schicht gebundenen Antikörper vorgängig mit dem photolytisch aktivierbaren CrosslinkingReagenz modifiziert werden. Die Modifikation der Proteinschicht mit diesem Reagenz würde nämlich die Aktivität dieser Schicht für das Binden des Fc-Teils der Antikörper drastisch verringern. Ebenso würde, wie oben erwähnt, bei einer chemischen Modifikation der zu immobilisierenden Antikörper durch ein Binden des Reagenzes in Antigen-erkennenden Bereichen die Aktivität für die Antigen/Hapten-Erkennung bzw. durch eine Addition am Fc-Teil die Erkennung dieses Fc-Teils durch das auf der Oberfläche immobilisierte Protein beeinträchtigt.

Die chemische Modifikation der immobilisierten Proteine bzw. der zu immobilisierenden Antikörper mit dem photolytisch aktivierbaren Crosslinking Reagenz wird dadurch umgangen, dass eine dritte Molekülart als Trägermoleküle (Hilfsmoleküle) für das Photoreagenz eingesetzt wird. Diese Trägermoleküle, welche unten noch weiter spezifiziert werden, werden extern mit dem Crosslinking-Reagenz so zur Reaktion gebracht, dass anschliessend mehrere photoaktivierbare Gruppen kovalent an ein einzelnes Trägermolekül gebunden sind. Die Trägermoleküle werden in dieser chemisch modifizierten Form entweder mit den Antikörper-ausrichtenden Proteinen auf der Wandleroberfläche immobilisiert oder mit den zu immobilisierenden Antikörpern an die Proteinoberfläche coadsorbiert. Es wurde nämlich überraschenderweise gefunden, dass sowohl die Coimmobilisierung derartiger Trägermoleküle mit den ausrichtenden Proteinen, als auch die Coadsorption von derartigen Trägermolekülen so geführt werden können, dass dadurch die Adsorption der Antikörper an die kovalent immobilisierte Proteinschicht nicht gestört wird.

Die gemeinsamen Eigenschaften, welche an diese Trägermoleküle gestellt werden müssen, sind:

i) eine hohe Wasserlöslichkeit,

ii) die Anwesenheit mehrerer funktioneller Gruppen für die Umsetzung mit dem photolytisch aktivierbaren Reagenz. Vernetzung kann nur erfolgen wenn mehrere aktivierbare Gruppen an einem Molekül vorhanden sind.

iii) die Anwesenheit funktioneller Gruppen für das kovalente Verknüpfen mit der Festphase bei der Coimmobilisierung,

iiii) bzw. für die Coadsorption Molekülbereiche, die mit den mit Fc-Teil erkennenden Proteinen bedeckten Oberflächen eine für die Adsorption genügend hohe Wechselwirkung eingehen.

Diese oben erwähnten Eigenschaften werden erfüllt durch Biomoleküle wie z.B. Albumine, Polysaccharide etc. oder durch wasserlösliche synthetische Polymere. Die Verwendung von geeigneten Biomolekülen hat den Vorteil, dass dadurch die Oberflächen bezüglich einer unspezifischen Adsorption sehr unempfindlich werden. Die Tatsache, dass eine effiziente Vernetzung nur erfolgt, wenn diese Trägermoleküle mehrere photoaktivierbare Gruppen enthalten, unterstreicht die Wichtigkeit der oben aufgestellten Forderung nach wasserlöslichen (hydrophilen), bifunktionellen Reagenzien, in welchen der photoaktivierbare Molekülteil wasserlöslich ist. Wäre dies nicht gewährleistet, würde durch die Modifikation die Wasserlöslichkeit der Trägermoleküle vermindert.

Die Coimmobilisierung dieser Trägermoleküle ist schematisch in Figur 1 dargestellt.

Zuerst werden die ausrichtenden Proteine (z.B. Protein A) und die Trägermoleküle (z.B. BSA) coimmobilisiert, wobei diese Trägermoleküle schon die photoaktivierbaren Crosslinking-Reagenzien (L) tragen (Fig. 1a).

Für die Coimmobilisierung dieser Trägermoleküle wird, wenn sie gleichzeitig mit der Immobilisierung der ausrichtenden Proteine durchgeführt wird, dasselbe Verfahren wie für die Immobilisierung der ausrichtenden Proteine verwendet. Das Konzentrationsverhältnis an der Oberfläche wird über die Wahl eines geeigneten Konzentrationsverhältnisses der beiden Molekülarten in Lösung eingestellt.

Dann erfolgt die Adsorption des Antikörpers (= biologisch erkennendes Molekül) dargestellt in Fig. 1b.

Die Vernetzung der Proteine untereinander und mit der Oberfläche durch photolytische Aktivierung des Crosslinking Reagenzes (L) wird in Fig. 1c durch Verbindungslinien symbolisiert.

Bezüglich der Art der Coadsorption lassen sich die Trägermoleküle in zwei Klassen aufteilen.

Eine erste Klasse umfasst Trägermoleküle, welche unspezifisch an diese Oberfläche adsorbieren (z.B. Albumine, Polysaccharide, wasserlösliche synthetische Polymere) und dabei hauptsächlich jene Teile der Oberfläche belegen, welche nicht mit den Fc-Teil erkennenden Proteinen belegt sind. Diese Klasse von Trägermolekülen wird bevorzugt vor der Adsorption der erkennenden Antikörper auf die Oberfläche adsorbiert.

Figur 2 zeigt schematisch das gerichtete kovalente Immobilisieren nativer Antikörper bei unspezifischer Adsorption der Trägermoleküle auf freie Bindungsplätze auf der Oberfläche.

Fig. 2a zeigt die kovalent immobilisierten, ausrichtenden Proteine (z.B. Protein A). In Fig. 2b ist die unspezifische Adsorption der Trägermoleküle (z.B. BSA) durch mit L-markierte Kreise dargestellt, die sich auf freien Plätzen zwischen den kovalent immobilisierten ausrichtenden Proteinen befinden. Danach werden die Antikörper gerichtet adsorbiert (Fig. 2c). Die Verbindungslinien zeigen wieder die Vernetzung der Proteine untereinander und mit der Oberfläche (Fig. 2d).

Eine zweite Klasse umfasst Trägermoleküle, welche spezifisch Fc-Bindungsstellen auf der kovalent immobilisierten Proteinschicht belegen. Typische Vertreter dieser Klasse sind Fc-Fragmente von Antikörpern. Diese Trägermoleküle können gleichzeitig mit den zu immobilisierenden Antikörpern oder erst nach deren Adsorption auf die Oberfläche adsorbiert werden. Im ersten Fall muss das Konzentrationsverhältnis von Trägermolekülen (z.B. Fc-Fragment) und erkennenden Antikörpern sehr genau optimiert werden, damit nicht zu viele Bindungsplätze durch Hilfsproteine belegt werden. Eine derartige Belegung der Fc-Bindungsstellen auf der Oberfläche mit Hilfsproteinen würde zu einer Verringerung der Konzentration an Antigen-erkennenden Antikörpern führen und dadurch die Empfindlichkeit und den dynamischen Bereich des Sensors drastisch verringern. Im Falle einer nachfolgenden Adsorption der Trägerproteine (z.B. des Fc-Fragmentes) werden durch diese primär Bindungsstellen abgesättigt, welche aus sterischen Gründen für die grösseren Antikörpermoleküle nicht zugänglich waren. Diese mit Crosslinking-Reagenz modifizierten Trägerproteine haben aber auch die Möglichkeit adsorbierte Antigen-erkennende Antikörper von der Oberfläche zu verdrängen. Da diese Verdrängung ein langsamer Prozess ist, kann er über die Inkubationszeit mit Hilfsproteinen sehr einfach kontrolliert werden.

Figur 3 zeigt schematisch das gerichtete kovalente Immobilisieren nativer Antikörper bei spezifischer Adsorption der Trägermoleküle auf Bindungsplätzen der ausrichtenden Proteine.

Fig. 3a zeigt ebenfalls die kovalent immobilisierten, ausrichtenden Proteine (z.B. Protein A).

In Fig. 3b wird die spezifische Coadsorption von Trägermolekülen (z.B. Fc-Teile von Antikörpern) durch Balken dargestellt, die mit L markiert sind und an den ausrichtenden Proteinen haften. Gleichzeitig werden die Antikörper

adsorbiert.

Die Vernetzung der Proteine untereinander und mit der Oberfläche wird durch die Verbindungslinien angedeutet (Fig. 3c).

Nach der Coadsorption von zu immobilisierenden Antikörpern und den mit Crosslinking-Reagenz modifizierten Trägerproteinen (bzw. der Coimmobilisierung der Letzteren mit dem ausrichtenden Protein) können die Proteine auf der Oberfläche durch Bestrahlung untereinander vernetzt werden. Auch hier ist es wichtig, das der photoaktivierbare Teil des bifünktionellen Crosslinking-Reagenzes sehr gut wasserlöslich ist. Nur durch diese hohe Wasserlöslichkeit ist gewährleistet, dass die aktivierte Gruppe (Nitrene) in die Lösung hinaus ragt und dadurch bevorzugt ein intermolekulares (nicht ein intramolekulares) Crosslinking erfolgt. Bei diesem photolytisch induzierten Crosslinking soll die Bestrahlungszeit möglichst kurz gehalten werden, um eine Zerstörung der Antikörper-Aktivität durch photolytisch induzierte Nebenreaktionen (so z.B. Photooxidation) gering zu halten.

Nachfolgend wird ein Beispiel für die photochemische Immobilisierung von anti HBsAg - (hepatitis B surface antigen ) - Antikörper gegeben.

1. Silanisierung der Sensoroberfläche

Die Silanisierung der Sensoroberfläche erfolgt in an sich bekannter Art und Weise während 15 Minuten in einer verdünnten Lösung von Octenyltrichlorsilan (0.5 %) in Hexadecan. Danach wird die Sensoroberfläche der Reihe nach gründlich mit Hexadecan, Hexan und Ethanol gespült. Die endständigen Doppelbindungen auf der silanisierten Oberfläche werden in einer Lösung von 2.5 mM Kaliumpermanganat und 100 mM Natriumperjodat während mindestens 60 Minuten zu Carbonsäuregruppen oxidiert. Die Reaktion wird durch Eintauchen des Sensors in eine 100 mM Natriumthiosulfitlösung abgestoppt und die Sensoroberfläche danach wiederum gründlich in Wasser und Ethanol gewaschen. Bei diesem Verfahren werden monomolekulare Zusatzschichten von hoher Qualität erhalten.

2. Immobilisierung von Protein A

Die Carbonsäuregruppen auf der Sensoroberfläche werden zuerst aktiviert und anschliessend zu N-Hydroxysuccinimidester umgesetzt. Die Aktivierung erfolgt in einer Lösung von 5% Chlorameisensäureethylester und 4% Pyridin in Methylenchlorid unter Schutzgas während 1 Stunde. Die Umwandlung dieser aktivierten Carbonsäuregruppen zu N-Hydroxysuccinimidesterfunktionen erfolgt durch Inkubation der Sensoroberfläche in einer Lösung von 500 mM N-Hydroxysuccinimid in Pyridin. Danach wird der Sensor mit Ethanol und Wasser gereinigt und anschliessend getrocknet.

Protein A wird auf der Sensoroberfläche immobilisiert, indem der Träger während 1 Stunde in einer Lösung dieses Proteins (0.1 mg/ml) in 100 mM Natriumcitratpuffer pH 4.8 inkubiert wird . Danach wird er mit Natriumcitratpuffer und Wasser gespült.

3. Adsorption von mit photoaktivierbaren Reagenz modifiziertem BSA

Auf einer derart präparierten Sensoroberfläche kann zusätzlich zum immobilisierten Protein A noch eine gewisse Menge BSA adsorbiert werden. Dieses BSA wird zuvor mit einem photoaktivierbaren Reagenz modifiziert. Dazu werden 10ml einer Lösung von BSA (10 mg/ml) in 1 M Ammoniumsulfatpuffer pH 9.0 vorgelegt und mit einer Lösung vom 7.5 mg 6-(p-Azidobenzol-sulfonylamino)capronsäure-N-hydroxysuccinimidester in 100 µl DMSO versetzt. Die Modifizierungsreaktion dauert 15 Minuten. Anschliessend wird die Sensoroberfläche mit dieser BSA-Lösung während 30 Minuten in Kontakt gebracht.

4. Adsorption der Antikörper und deren photochemische Verankerung

5 mg/ml Antikörper werden in 1 M Ammoniumsulfatpuffer pH 9.0 gelöst. In dieser Lösung wird die Sensoroberfläche während 30 Minuten inkubiert, so dass Antikörper auf das Protein A adsorbieren können. Danach wird die Sensoroberfläche zuerst mit dem gleichen Ammomumsulfatpuffer gespült und anschliessend wieder damit überschichtet. Danach erfolgt die Belichtung mit einer Quecksilberdampflampe während 30 Sekunden. Dieses Verfahren führt zu einer photochemischen Vernetzung von Protein A, BSA und dem Fc-Teil des Antikörpers. Die Bindungsstellen der Antikörper bleiben dabei intakt. Eine solche Sensoroberfläche kann nun physiologischen Pufferbedingungen ausgesetzt werden ohne dass Desorption der Antikörper stattfindet.

Die photoaktivierbaren Reagenzien werden wie folgt hergestellt.

Allgemeine Bemerkungen

Bei allen Verbindungen die eine Azidogruppe enthalten, wurde unter Lichtausschluss nachgerührt.
HPLC wurden bei einem Fluss 1ml/min. ausgeführt und im UV bei 254nm detektiert.

Beispiel 1

41mg N-(p-Azidobenzolsulfonyl)N'-(3-carboxypropionyl)cystamin wurden 5 Stunden gerührt mit 1ml Thionylchlorid und anschliessend im WSV eingeengt. Das rohe Säurechlorid wurde in 5ml THF gelöst und mit 14mg N-Hydroxysuccinimid als Lösung in 1ml Pyridin versetzt. Es wurde 2 Stunden gerührt und anschliessend im HV eingeengt. Man erhielt 68mg N-(p-Azidobenzolsulfonyl)N'-(3-succinimidyloxycarbonylpropionyl)cystamin als Pyridiniumsalz.
Das verwendete Ausgangsmaterial wurde wie folgt hergestellt:
2,2g Cystamindihydrochlorid wurden in 20ml Wasser gelöst und mit NaOH auf pH10 eingestellt. In dieser Lösung wurden 2,1g p-Azidobenzolsulfochlorid suspendiert und 5 Stunden bei Raumtemperatur gerührt. Das ausgefallene N-(p-Azidobenzolsulfonyl)cystamin wurde mit 2g Bernsteinsäureanhydrid umgesetzt und über Nacht gerührt. Die dabei entstandene Lösung wurde mit HCl angesäuert, anschliessend filtriert und mit Wasser gewaschen. Der Rückstand wurde bei RT im HV getrocknet und ergab 1,53g N-(p-Azidobenzolsulfonyl)-N'-(3-carboxypropionyl)cystamin. Das IR zeigte Banden bei 3283(Amid-NH), 2134(Azid), 1714(Säurecarbonyl), 1650(Amid), 1589+1547(Aromat), 1284(COOH), 1328+1180(Arylsulfonyl), 839(p-disubst. Benzol). DC(Kieselgel-$NH_3$ conc./EtOH = 1%) Rf = 0,7. Smp:Zers. bei 163°.

Beispiel 2

0,5g ε-(p-Azidobenzolsulfonyl)aminocapronsäure wurden 5 Stunden gerührt mit 5ml Thionylchlorid und anschliessend im WSV eingeengt. Das rohe Säurechlorid wurde in 5ml THF gelöst und mit 348mg N-Hydroxysuccinimid als Lösung in 5ml Pyridin versetzt. Es wurde 2 Stunden gerührt und anschliessend im HV eingeengt. Man erhiehlt 1,09mg ε-(p-Azidobenzolsulfonylamino)capronsäure N-Hydroxysuccinimidester als Pyridiniumsalz.
Das verwendete Ausgangsmaterial wurde wie folgt hergestellt:
In einer Lösung von 8,8g $NaHCO_3$ in 100ml Wasser wurden 4,57g ε-Aminocapronsäure gelöst. Darin wurden 7,6g p-Azidobenzolsuifonsäurechlorid suspendiert und über Nacht gerührt. Aus der entstandenen Lösung wurde mit HCl gefällt, filtriert und mit Wasser gewaschen. Es wurden nach dem Trocknen im HV bei RT 4,56g ε-(p-Azidobenzolsulfonylamino)capronsäure erhalten. Das IR zeigte die erwarteten Banden bei 2097(Azid), 1715(Säurecarbonyl), 1585+1400(Aromat), 1282(COOH), 1319+1153(Arylsulfonyl), 834 (p-disubst.Benzol).DC(Kieselgel-EtOAc/EtOH=3)Rf=0,55.
Smp=126-127°
Im Analogie hergestellte ε-(p-Azidobenzolsulfonylamino)capronsäure zeigte im DC dasselbe Laufverhalten und einen Smp von 132-133°. Es kann gemäss Beispiel 6 in ε-(p-Azidobenzolsulfonylamino)capronsäure N-Hydroxysuccinimidester übergeführt werden.

Beispiel 3

31mg ε-(p-Azidobenzolsulfonyl)aminocapronsäure wurden 5 Stunden gerührt mit 1ml Thionylchlorid und anschliessend im WSV eingeengt. Das rohe Säurechlorid wurde in 5ml THF gelöst und mit 22mg N-Hydroxysuccinimid-2-sulfonsäure als Lösung in 1ml Pyridin versetzt. Es wurde 2 Stunden gerührt und anschliessend im HV eingeengt. Man erhiehlt 58mg ε-(p-Azidobenzolsulfonyl)aminocapronsaure (N-Hydroxysuccinimid-2-sulfonsäure)ester als Dipyridiniumsälz.
Das verwendete Ausgangsmaterial wurde gemäss Beispiel 2 hergestellt.

Beispiel 4

550mg ε-(p-Azidoberzoyl)aminocapronsäure wurden in 5ml THF gelöst und mit 0,5ml Thionylchlorid versetzt. Es wurde 5 Stunden gerührt und anschliessend eingeengt. Das rohe Säurechlorid wurde in 5ml THF gelöst, mit 251mg 2-(2-Aminoethyldithiopyridin) in 2ml Pyridin versetzt und 2 Stunden nachgerührt. Es wurden 20g Eis zugegeben, mit NaOH auf pH6 eingestellt und mit 6g NaCl versetzt. Es wurde 1 Stunde gerührt, anschliessend filtriert, mit gesättigter Kochsalzlösung gewaschen und im HV (RT) getrocknet. DC(Kieselgel-HCl conc./EtOH=1%) Rf=0,2; DC(Kieselgel-EtOAc) Rf=0,6.

Beispiel 5

280mg ε-(p-Azidobenzoyl)aminocapronsäure N-Hydroxysuccinimidester wurde in 5ml THF gelöst und zu einer Lösung von 140mg Hydrazinium monochlorid in 20ml Wasser zugetropft. Es wurde mit Ueberschuss Soda basisch eingestellt und anschliessend filtriert. Der Rückstand wurde mit Wasser gewaschen und im HV getrocknet. Man erhielt 305mg ε-(p-Azidobenzoyl)aminocapronsäurehydrazid. DC(Kieselgel-HCl conc./EtOH=1%) Rf=0,7; DC(Kieselgel-$NH_3$ conc./EtOH=1%) Rf=0,8.

Beispiel 6

100mg 6-(3-Azido-5-sulfobenzoylamino)hexansäure (0,28 mmol) wurden in 2ml trockenem THF bei RT gelöst, mit 0,7ml Thionylchlorid durch Zutropfen unter Rühren versetzt und anschliessend über Nacht bei RT gerührt (Magnetrührer). Dann wurde das überschüssige Thionylchlorid zusammen mit dem Lösungsmittel am Wasserstrahlvakuum abgezogen ($CaCl_2$-Rohr) und zur vollständigen Entfernung des Thionylchlorids noch 2 mal mit je 2ml THF abrotiert (Magnetrührer, Wasserstrahlvakuum). Der gelbliche Rückstand wurde mit 2ml THF aufgenommen, die Lösung mit 35mg N-Hydroxysuccinimid in 1ml THF versetzt und nach der Zugabe von 200mg fein pulverisiertem Natriumhydrogencarbonat während 24 Stunden bei RT gerührt. Nach Abtrennung des Bodenkörpers wurde die gelbliche Lösung am Wasserstrahlvakuum bei RT eingeengt; durch Zugabe von Essigester wurde das Produkt, 6-(3-Azido-5-sulfobenzoylamino)hexansäure-N-hydroxysuccinimidester, als gelbes Pulver (ca 70 mg) abgeschieden und am HV bei RT getrocknet. IR (KBr;$cm^{-1}$): 3420(Amid-NH), 2114(Azid), 1737(Estercarbonyl), 1658 (Amidcarbonyl), 1589(Aromat), 1541(Amid-2-Bande), 1195(Sulfonat). DC(Kieselgel/Ethanol-$NH_3$): Rf-Produkt=0,8, Rf-Edukt=0,6, Rf-Hydroxysuccinimid=0,05.

Das verwendete Ausgangsmaterial wurde wie folgt hergestellt:

14,6g 3-Nitro-5-sulfobenzoesäuremononatriumsalz wurden in 300ml Pyridin gelöst und 2 Stunden gerührt, dann wurden 2,2ml Oxalylchlorid zugetropft. Nach 1 Stunde wurden 16,6g ε-aminocapronsäure-benzylester als Lösung in 100ml THF zugetropft und danach über Nacht gerührt. Es wurde am Rotationsverdampfer eingeengt und anschliessend mit 100g stark saurem Ionenaustauscher in 100ml Wasser 50 Stunden gerührt. Es wurde filtriert, mit Wasser gewaschen und dann das Filtrat eingeengt. Der Rückstand wurde in 30% Methanol/Wasser aufgenommen, mit 21g Tetraethylammoniumbromid versetzt und mit Natronlauge auf pH 6,5 eingestellt. Die erhaltene Lösung wurde an 500g silanisiertem Kieselgel (RP2) mit 30% Methanol chromatographiert. Es wurden 8,6 g ε-(3-Nitro-5-Sulfobenzoylamino)capronsäurebenzylester als Tetraethylammoniumsalz erhalten, welches mit 100g Kationenaustauscher in Wasser verrührt wurde und nach filtrieren, mit Wasser waschen und einengen des Filtrates die entsprechende freie Sulfonsäure ergab. DC(Kieselgel-HOAc/EtOAc=20%) Rf=0,7 (Edukt=0,4); DC(Kieselgel-$NH_3$/EtOH=1%) Rf=0,8 (Edukt=0,7); DC(RP18-EtOH/$Et_4N^+$(0,01M) Phosphatpuffer(0,1M; pH 6,5)=60%) Rf=0,4 (Edukt=0,8). HPLC(RP18-MeCN/$Et_4N^+$(0,01M)Phosphatpuffer(0,1M; pH 6,5)=30%) $t_R$=5,9 min. (Edukt=1,3min.)

1,32g 6-(3-Nitro-5-sulfobenzoylamino)hexansäure-benzylester wurden gelöst in 30ml Ethanol/Wasser=60%, 100mg Pd/C 10% zugefügt und unter Rühren (Magnetrührer) hydriert: Die gesamte Wasserstoffaufnahme (1,5 Std.) betrug 235 ml. Die DC-Kontrolle zeigt kein Ausgangsmaterial mehr. Im DC ist das Amin praktisch am Start. Der Katalysator wurde abfiltriert und die Lösung am RV zur Trockene eingeengt. Die IR-Kontrolle der Substanz Zeigt das vollständige Fehlen der ursprünglichen Nitrogruppe und der Benzylgruppe. Die so erhaltene 6-(3-Amino-5-sulfobenzoylamino)hexansäure wurde direkt weiterverwendet für die Azidherstellung

ca. 1,0g 6-(3-Amino-5-sulfobenzoylamino)hexansäure = ca. 3,0 mmol wurden in 15ml Wasser und 3ml THF gelöst, 1ml HCl conc. wurde zugefügt und die Lösung wurde auf 0° gekühlt. Dann wurden 210mg Natriumnitrit in 1,5ml Wasser zugetropft bei 0°. Nach beendetem Zutropfen fiel ein feiner Niederschlag aus (Diazoniumsalz); Es wurde 1 Stunde bei RT. gerührt. Dann wurden 208mg Natriumazid in 1ml Wasser langsam zugetropft: (Gasentwicklung, Schäumen; Suspension geht in Lösung). Der Austausch mit Natriumazid läuft rasch; Das DC zeigt nach 1 Stunde nur das Azid, kein Amin mehr,

DC mit Kieselgel-Ethanol/HCl: Rf(Azid): ca. 0,6; Rf(Amin): ca. 0,5; Amin fluoresziert, Azid absorbiert. Azidfleck wird unter UV-Licht braun. Azidlösung unter Rühren bei RT. am HV. vollständig eingeengt. Pulvrig-kristallin; gelblich. IR-Kontrolle: Alle geforderten IR-Banden für 6-(3-Azido-5-sulfobenzoylamino)hexansäure korrekt vorhanden.

Beispiel 7

Es wurde β-(3-Azido-5-sulfobenzoylamino)propionsäure analog Beispiel 6 zum N-Hydroxysuccinimidester umgesetzt.

Das verwendete Ausgangsmaterial wurde wie folgt hergestellt:

14,6g 3-Nitro-5-sulfobenzoesäure mononatriumsalz wurden in 300ml Pyridin gelöst und 2 Stunden gerührt, dann wurden 2,2ml Oxalylchlorid zugetropft. Nach 1 Stunde wurden 13,6g β-Alanin tert.butylester zugegeben und

danach über Nacht gerührt. Es wurde am Rotationsverdampfer eingeengt und anschliessend mit 100g stark saurem Ionenaustauscher in 100ml Wasser 16 Stunden gerührt. Es wurde filtriert, mit Wasser gewaschen und dann das Filtrat eingeengt. Der Rückstand wurde in 30% Methanol/Wasser aufgenommen, mit 21g Tetraethylammoniumbromid versetzt und mit Natronlauge auf pH 6,5 eingestellt. Die erhaltene Lösung wurde an 1kg silanisiertem Kieselgel (RP2) mit 30% Methanol chromatographiert. Es wurden ca. 6g β-(3-Nitro-5-sulfobenzoyl)aminopropionsäure als Tetraethylammoniumsalz erhalten, welches mit 100g Kationenaustauscher in Wasser verrührt wurde und nach filtrieren, mit Wasser waschen und einengen des Filtrates die entsprechende freie Sulfonsäure ergab. DC(Kieselgel-HOAc/EtOAc) Rf=0,5 (Nebenprodukt:Diamid=0,9).

Die Umwandlung der Nitrogruppe via Reduktion zur Aminogruppe und Sandmeyer-Reaktion zur Azidgruppe erfolge in Analogie zu Beispiel 6.

Beispiel 8

8,6g N-(3-Azido-5-sulfobenzoyl)-N'-(3-carboxypropionyl)ethylendiamin wurden in 50ml THF gelöst, mit 10ml Thionylchlorid versetzt und 5 Stunden gerührt. Es wurde eingeengt und der Rückstand (Rohsäurechlorid) in 50ml THF gelöst, nacheinander mit 2g N-Hydroxysuccinimid als Lösung in 10ml THF und 10g Natriumbicarbonat versetzt. Es wurde über Nacht gerührt. filtriert, der Rückstand mit THF gewaschen, anschliessend das Filtrat eingeengt und im HV bei RT getrocknet. DC(Kieselgel-$NH_3$ conc./EtOH=1%) Rf=0,8.

Das verwendete Ausgangsmaterial wurde wie folgt hergestellt:

12g 3-Nitro-5-sulfobenzoesäure mononatriumalz wurden 2 Stunden gerührt mit 200ml Pyridin und dann mit 8,8g Thionylchlorid versetzt. Nach 1 Stunde wurden 24g Ethylendiamin zugetropft und über Nacht gerührt. Dann wurde eingeengt, der Rückstand in THF aufgenommen nacheinander 10g Bernsteinsäureanhydrid und 20g Soda zugegeben und wieder über Nacht gerührt. Es wurde filtriert, mit THF gewaschen, das Filtrat wurde eingeengt und der Rückstand in die nächste Stufe eingesetzt. 3310+3255(Amin), 1730(Säurecarbonyl), 1640+1506(Aromat), 1640+1539(Amid), 1296(Arylsulfonyl), DC(Kieselgel-HCl conc./EtOH=1%) Rf=0,5; DC(Kieselgel-$NH_3$ conc./EtOH=1%) Rf=0,6. HPLC (10%MeCN-RP18)$t_R$=1,7 min. ($t_R$-Zwischenprodukt=3,3min./$t_R$-Edukt=3min.).

N-(3-Amino-5-sulfobenzoyl)-N'-(3-carboxypropionyl)ethylendiamin wurde in 12ml Salzsäure (37%) und 100ml Wasser gelöst, bei 0° tropfenweise mit Natriumnitritlösung (4N) versetzt und 1 Stunde bei dieser Temperatur nachgerührt (zeigte Reaktion mit KJ/Stärke und mit alkalischer 2-Naphthol-Lösung). Dann wurden 13g Natriumazid langsam zugegeben, 5 Stunden bei RT nachgerührt, anschliessend wurden 20g NaCl zugegeben, 1 Stunde gerührt und filtriert. Der Rückstand wurde mit gesättigter Kochsalzlösung gewaschen und im HV bei RT getrocknet. Das Rohprodukt wurde im IR charakterisiert und direkt in die nächste Stufe eingesetzt. 2121(Azid), 1736(Säurecarbonyl), 1507(Aromat), 1640+1507(Amid), 1180(Arylsulfonyl), 857(p-disubst.Benzol)

Beispiel 9

Es wurden 15g 2-(p-Azidobenzoylaminomethyl)pyridin in 50ml THF gelöst und bei -20° mit 50g 2-Bromessigsäurebromid versetzt und über Nacht bei dieser Temperatur stehen gelassen. Es wurde eingeengt und in 50ml THF aufgenommen. Es wurden 11,5g N-Hydroxysuccinimid zugegeben, über Nacht bei RT gerührt und anschliessend eingeengt. Man erhielt dabei N-(succinimidyloxycarbonylmethyl)-2-(p-Azidobenzoylaminomethyl)pyridin.

Oder es wurden 15g 2-(p-Azidobenzoylaminomethyl)pyridin in 500ml Wasser gelöst und mit 10g 2-Chloressigsäure versetzt und über Nacht gerührt. Es wurden 100g NaCl zugegeben, 1 Stunde gerührt, filtriert, der Rückstand mit gesättigter Kochsalzlösung gewaschen und im HV getrocknet. Es wurde N-(Carboxymethyl)-2-(Azidobenzoylaminomethyl)-pyridin erhalten, welches im IR folgende Banden zeigte: 3427+3283(Amid-NH), 2121(Azid), 1633+1565(Amid) 1499+1601(Aromat), 859(p-disubst.Benzol), 710+763(monosubst.Benzol).

Es kann gemäss Beispiel 6 in das bereits oben beschriebene N-(succinimidyloxycarbonylmethyl)-2-(p-Azidobenzoylaminomethyl)pyridin überführt werden.

Das verwendete Ausgangsmaterial wurde wie folgt hergestellt:

14,3g p-Azidobenzoesäure wurde suspendiert in 100ml Thionlylchlorid und 5 Stunden gerührt (dabei entstand eine Lösung). Es wurde eingeengt, der Rückstand in 50ml THF gelöst und 30ml 2-Aminomethylpyridin zugegeben und 2 Stunden bei RT gerührt. Dann wurde auf 200g Eis gegossen, mit NaOH neutralisiert, filtriert, mit Wasser gewaschen und im HV getrocknet. Im IR zeigten sich Banden bei 3306(Amid-NH), 2125(Azid), 1626+1547(Amid), 1500+1603(Aromat), 852(p-disubst.Benzol), HPLC(RP18/30-100%MeCN in 20 min.) $t_R$=8min. ($t_R$-Edukt=10min.). DC(Kieselgel-HOAc/EtOAc=20%) Rf=0,2.

Beispiel 10

5-Azido-isophthalsäuredichlorid wurde aus 2,5g 5-Azido-isophthalsäure frisch hergestellt und roh umgesetzt mit einer Lösung von 5,2g ε-Aminocapronsäure in 100ml Wasser und 10g Soda. Nach 5 Stunden Rühren wurde die entstandene Lösung mit HCl sauer eingestellt, anschliessend filtriert und mit Wasser gewaschen. Der Rückstand bestehend aus 3,5-Di(6-Capronyl-amino)azidobenzol wurde getrocknet und im IR charakterisiert: 3460(Amid-NH), 2121(Azid), 1717+1559(Amid), 1599+1631(Aromat). Es wurden gemäss Beispiel 6 die Carbonsäuregruppen aktiviert.

**Patentansprüche**

1. Biologisch erkennende Schichten, die auf einer Festphase kovalent immobilisiert sind, wobei die Oberfläche der Festphase mit einer organischen Zusatzschicht versehen ist, die die für eine Proteinimmobilisierung geeigneten funktionellen Gruppen in hoher Dichte besitzt, und die aus biologisch erkennenden Molekülen bestehen, die den Analyten erkennende und bindende Molekülbereiche und den Analyten nicht erkennende und bindende Molekülbereiche aufweisen, wobei die biologisch erkennenden Moleküle in einer Vorzugsrichtung derart kovalent immobilisiert sind,

   (a) dass die den Analyten erkennenden und bindenden Molekülbereiche von der Oberfläche der Festphase abgewandt sind und nicht durch die kovalente Bindung verändert sind.

   (b) dass die den Analyten nicht erkennenden Molekülbereiche an eine die erkennenden Moleküle ausrichtende Schicht gebunden sind, welche spezielle Bindungstellen für diese gerichtete Adsorption der biologisch erkennenden Moleküle aufweist und aus ausrichtenden Molekülen besteht, die an der organischen Zusatzschicht immobilisiert sind, und

   (c ) dass die biologisch erkennenden Moleküle untereinander und mit den ausrichtenden Molekülen über chemisch modifizierte Trägermoleküle quervernetzt sind, wobei die Quervernetzung unter Verwendung von Trägermolekülen photochemisch erfolgt ist, welche Trägermoleküle mehrere photoaktivierbare Gruppen pro Molekül tragen und eine hohe Wasserlöslichkeit, insbesondere im photoaktivierbaren Teil des Moleküls, aufweisen.

2. Biologisch erkennende Schichten nach Anspruch 1, dadurch gekennzeichnet, dass die Trägermoleküle Biomoleküle sind.

3. Biologisch erkennende Schichten nach Anspruch 1 oder 2, dadurch gekennzeichnet, dass die biologisch erkennende Moleküle Antikörper sind, und dass die ausrichtende Schicht aus ausrichtenden Proteinen besteht, welche eine spezifische Affinität zum Fc-Teil von Antikörpern besitzen.

4. Biologisch erkennende Schichten nach Anspruch 3, dadurch gekennzeichnet, dass die Trägermoleküle hauptsächlich jene Teile der Oberfläche belegen, welche nicht mit den Fc-Teil erkennende ausrichtenden Proteinen belegt sind.

5. Biologisch erkennende Schichten nach Anspruch 3, dadurch gekennzeichnet, dass die Trägermoleküle Spezifisch Fc-Bindungsstellen auf den ausrichtenden Proteinen belegen.

6. Biologisch erkennende Schichten nach einem der Ansprüche 3-5, dadurch gekennzeichnet, dass die ausrichtenden Proteine pro Molekül mehrere Bindungsstellen für den Fc-Teil von Antikörpern besitzen.

7. Biologisch erkennende Schichten nach einem der Ansprüche 3-6, dadurch gekennzeichnet, dass die ausrichtenden Proteine einen wesentlich geringeren Raumbedarf als die Antikörper aufweisen.

8. Verfahren zur Herstellung biologisch erkennender Schichten nach einem der vorangegangenen Ansprüche, dadurch gekennzeichnet, dass vor der Quervernetzung die gerichtete Adsorption der biologisch erkennenden Moleküle erfolgt, die dadurch erreicht wird, dass vor der Adsorption der biologisch erkennenden Moleküle auf der Oberfläche der Festphase durch chemische bzw. biochemische Behandlung Bindungsstellen geschaffen werden, welche den Analyten nicht erkennende Molekülbereiche der biologisch erkennenden Moleküle adsorptiv binden, wobei die Quervernetzung unter Verwendung von Trägermolekülen photochemisch erfolgt, welche Trägermoleküle mehrere photoaktivierbare Gruppen pro Molekül tragen und eine hohe Wasserlöslichkeit, insbesondere im photoaktivierbaren Teil des Moleküls, aufweisen.

9. Verfahren zur Herstellung biologisch erkennender Schichten nach Anspruch 8 , dadurch gekennzeichnet, dass die

Trägermoleküle vor, gleichzeitig mit oder nach der gerichteten Adsorption der den Analyten erkennenden Moleküle auf der Oberfläche der Festphase adsorbiert werden.

**10.** Verfahren zur Herstellung biologisch erkennender Schichten nach einem der Ansprüche 8 und 9, dadurch gekennzeichnet, dass die chemische Modifikation der Trägermoleküle unter Verwendung von gut wasserlöslichen bifunktionellen Reagenzien erfolgt.

**11.** Verfahren zur Herstellung biologisch erkennender Schichten nach Anspruch 10, dadurch gekennzeichnet, dass es sich bei den bifunktionellen Gruppen in den bifunktionellen Reagenzien um eine chemisch reaktive Gruppe und um eine photoaktivierbare Phenylazidogruppe handelt.

**12.** Verfahren zur Herstellung biologisch erkennender Schichten nach Anspruch 11, dadurch gekennzeichnet, dass die beiden funktionellen Gruppen über eine Spacergruppe miteinander verknüpft sind.

**13.** Verfahren zur Herstellung biologisch erkennender Schichten nach einem der Ansprüche 11 und 12, dadurch gekennzeichnet, dass es sich bei den wasserlöslich machenden Gruppen in den bifunktionellen Reagenzien um Sulfonsäure, Carbonsäure oder Derivate davon, um eine Hydroxylgruppe oder tertiäre Amino oder quartäre Ammoniumgruppen handelt.

**14.** Verfahren zur Herstellung biologisch erkennender Schichten nach einem der Ansprüche 11-13, dadurch gekennzeichnet, dass die wasserlöslich machenden Gruppen in den bifunktionellen Reagenzien entweder an die Spacergruppe oder an die photoaktivierbare Gruppe gebunden sind und bei der Umsetzung mit den Trägermolekülen nicht abgespalten werden.

## Claims

**1.** Biologically recognizing layers which are covalently immobilized on a solid phase, wherein the surface of the solid phase is provided with an organic additional layer which has suitable functional groups for protein immobilization in a high density and which is composed of biologically recognizing molecules which have molecular regions which recognize and bind the analyte and molecular regions which do not recognize and bind the analyte, wherein the biologically recognizing molecules are covalently immobilized in a preferred direction such that

(a) the molecular regions which recognize and bind the analyte face away from the surface of the solid phase and are not changed by the covalent bond;

(b) the molecular regions which do not recognize the analyte are bound to a layer which aligns the recognizing molecules and has special binding sites for this directed adsorption of the biologically recognizing molecules and is composed of aligning molecules which are immobilized on the additional organic layer, and

(c) the biologically recognizing molecules are cross-linked with one another and with the aligning molecules via chemically modified carrier molecules, wherein the cross-linking is carried out photochemically using carrier molecules and these carrier molecules carry several photo-activatable groups per molecule and have a high water-solubility especially in the photo-activatable part of the molecule.

**2.** Biologically recognizing layers as claimed in claim 1, wherein the carrier molecules are biomolecules.

**3.** Biologically recognizing layers as claimed in claim 1 or 2, wherein the biologically recognizing molecules are antibodies and the aligning layer is composed of aligning proteins which have a specific affinity for the Fc part of antibodies.

**4.** Biologically recognizing layers as claimed in claim 3, wherein the carrier molecules mainly occupy those parts of the surface which are not occupied by aligning proteins which recognize the Fc part.

**5.** Biologically recognizing layers as claimed in claim 3, wherein the carrier molecules occupy specifically Fc binding sites on the aligning proteins.

**6.** Biologically recognizing layers as claimed in one of the claims 3 to 5, wherein the aligning proteins have several binding sites per molecule for the Fc part of antibodies.

**7.** Biologically recognizing layers as claimed in one of the claims 3 to 6, wherein the aligning proteins have a substantially lower spatial requirement than the antibodies.

**8.** Process for the production of biologically recognizing layers as claimed in one of the previous claims, wherein the aligned adsorption of the biologically recognizing molecules takes place before cross-linking by creating binding sites on the surface of the solid phase by chemical or biological treatment before adsorption of the biologically recognizing molecules and these binding sites adsorptively bind molecular regions of the biologically recognizing molecules which do not recognize the analyte, wherein the cross-linking is carried out photochemically using carrier molecules which carry several photo-activatable groups per molecule and have a high water-solubility especially in the photo-activatable part of the molecule.

**9.** Process for the production of biologically recognizing layers as claimed in claim 8, wherein the carrier molecules are adsorbed onto the surface of the solid phase before, simultaneously with or after the aligned adsorption of the molecules which recognize the analyte.

**10.** Process for the production of biologically recognizing layers as claimed in one of the claims 8 and 9, wherein the carrier molecules are chemically modified using bifunctional reagents which dissolve readily in water.

**11.** Process for the production of biologically recognizing layers as claimed in claim 10, wherein the bifunctional groups in the bifunctional reagents are a chemically reactive group and a photo-activatable phenylazido group.

**12.** Process for the production of biologically recognizing layers as claimed in claim 11, wherein the two functional groups are linked via a spacer group.

**13.** Process for the production of biologically recognizing layers as claimed in one of the claims 11 and 12, wherein the water-solubilizing groups in the bifunctional reagents are sulphonic acid, carboxylic acid or derivatives thereof, a hydroxyl group or tertiary amino or quaternary ammonium groups.

**14.** Process for the production of biologically recognizing layers as claimed in one of the claims 11 to 13, wherein the water-solubilizing groups in the bifunctional reagents are either bound to the spacer group or to the photo-activatable group and are not cleaved off during the reaction with the carrier molecules.

**Revendications**

**1.** Couches à reconnaissance biologique, qui sont immobilisées de façon covalente sur une phase solide, la surface de la phase solide étant munie d'une couche additionnelle organique qui possède en une densité élevée les groupes fonctionnels appropriés pour l'immobilisation des protéines, et qui se composent de molécules à reconnaissance biologique, qui présentent des domaines moléculaires reconnaissant et liant les analytes et des domaines moléculaires ne reconnaissant ni ne liant les analytes, les molécules à reconnaissance biologique étant immobilisées de façon covalente dans une direction préférée de manière

(a) que les domaines moléculaires reconnaissant et liant les analytes soient opposés à la surface de la phase solide et ne soient pas modifiés par la liaison covalente,
(b) que les domaines moléculaires ne reconnaissant pas les analytes soient liés à une couche dirigeant les molécules de reconnaissance, qui présentent des endroits de liaison particuliers pour l' adsorption dirigée des molécules de reconnaissance biologique et qui se composent de molécules dirigeantes qui sont immobilisées sur la couche additionnelle organique, et
(c) que les molécules à reconnaissance biologique sont réticulées entre elles et avec les molécules dirigeantes par l'intermédiaire de molécules support modifiées, la réticulation s'effectuant par un moyen photochimique avec utilisation de molécules supports, ces molécules supports portant plusieurs groupes photoactivables par molécule et présentant une haute solubilité dans l'eau, en particulier dans la partie photoactivable de la molécule.

**2.** Couches à reconnaissance biologique selon la revendication 1, caractérisées en ce que les molécules support sont des biomolécules.

**3.** Couches à reconnaissance biologique selon la revendication 1 ou 2, caractérisées en ce que les molécules à reconnaissance biologique sont des anticorps, et en ce que la couche dirigeante est composé de protéines dirigeantes qui possèdent une affinité spécifique à la partie Fc des anticorps.

**4.** Couches à reconnaissance biologique selon la revendication 3, caractérisées en ce que les molécules support recouvrent principalement les parties de la surface qui ne sont pas recouvertes avec les protéines dirigeantes qui reconnaissent la partie Fc.

**5.** Couches à reconnaissance biologique selon la revendication 3, caractérisé en ce que les molécules support recouvrent spécifiquement des endroits de liaison à Fc sur les protéines dirigeantes.

**6.** Couches à reconnaissance biologique selon l'une des revendications 3-5, caractérisées en ce que les protéines dirigeantes possèdent par molécule plusieurs endroits de liaison pour la partie Fc des anticorps.

**7.** Couches à reconnaissance biologique selon l'une des revendications 3-6, caractérisées en ce que les protéines dirigeantes ont un besoin d'espace nettement plus faible que les anticorps.

**8.** Procédé de production de couches à reconnaissance biologique selon l'une des revendications précédentes, caractérisé en ce qu'avant la réticulation il se produit une adsorption dirigée des molécules à reconnaissance biologique, que l'on obtient en créant avant l'adsorption des molécules à reconnaissance biologique sur la surface de la phase solide, par un traitement chimique ou selon les cas biochimique, des endroits de liaison qui lient par adsorption les zones moléculaires des molécules à reconnaissance biologique qui ne reconnaissent pas l'analyte, la réticulation s'effectuant par un moyen photochimique avec utilisation de molécules support, ces molécules support portant plusieurs groupes photoactivables par molécule et présentant une haute solubilité dans l'eau, en particulier dans la partie photoactivable de la molécule.

**9.** Procédé de production de couches à reconnaissance biologique selon la revendication 8, caractérisé en ce que les molécules support sont adsorbées avant, en même temps que ou après l'adsorption dirigée des molécules reconnaissant l'analyte à la surface de la phase solide.

**10.** Procédé de production de couches à reconnaissance biologique selon l'une des revendications 8 et 9, caractérisé en ce que la modification chimique des molécules supports s'effectue avec utilisation de réactifs bifonctionnels bien solubles dans l'eau.

**11.** Procédé de production de couches à reconnaissance biologique selon la revendication 10, caractérisé en ce que, pour ce qui est des groupes bifonctionnels dans les réactifs bifonctionnels, il s'agit d'un groupe chimiquement réactif et d'un groupe phénylazido photoactivable.

**12.** Procédé de production de couches à reconnaissance biologique selon la revendication 11, caractérisé en ce que les deux groupes fonctionnels sont reliés entre eux par l'intermédiaire d'un groupe espaceur.

**13.** Procédé de production de couches à reconnaissance biologique selon l'une des revendications 11 et 12, caractérisé en ce que, pour ce qui est des groupes solubilisants dans l'eau dans les réactifs bifonctionnels, il s'agit d'un acide sulfonique, d'un acide carboxylique ou de leurs dérivés, d'un groupe hydroxyle ou de groupes amino tertiaires ou ammonium quaternaire.

**14.** Procédé de production de couches à reconnaissance biologique selon l'une des revendications 11-13 caractérisé en ce que des groupes solubilisants dans l'eau dans les réactifs bifonctionnels sont ou bien liés au groupe espaceur ou bien au groupe photoactivable et ne sont pas séparés lors de la réaction avec les molécules supports.

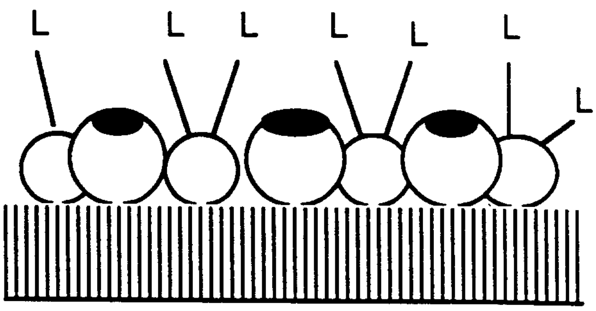


a)

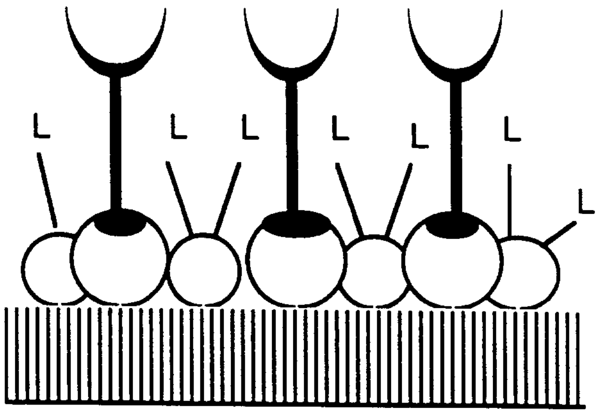


b)

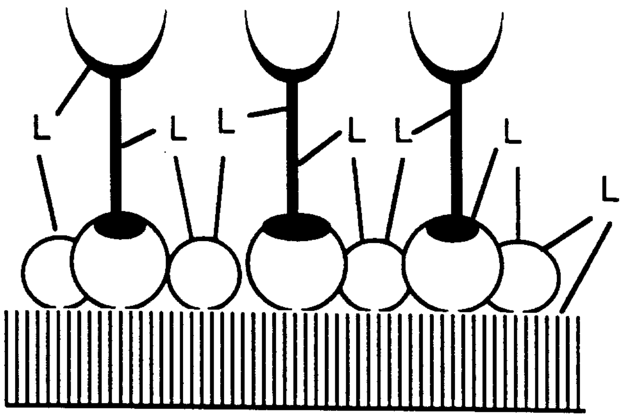


c)

**Fig. 1**

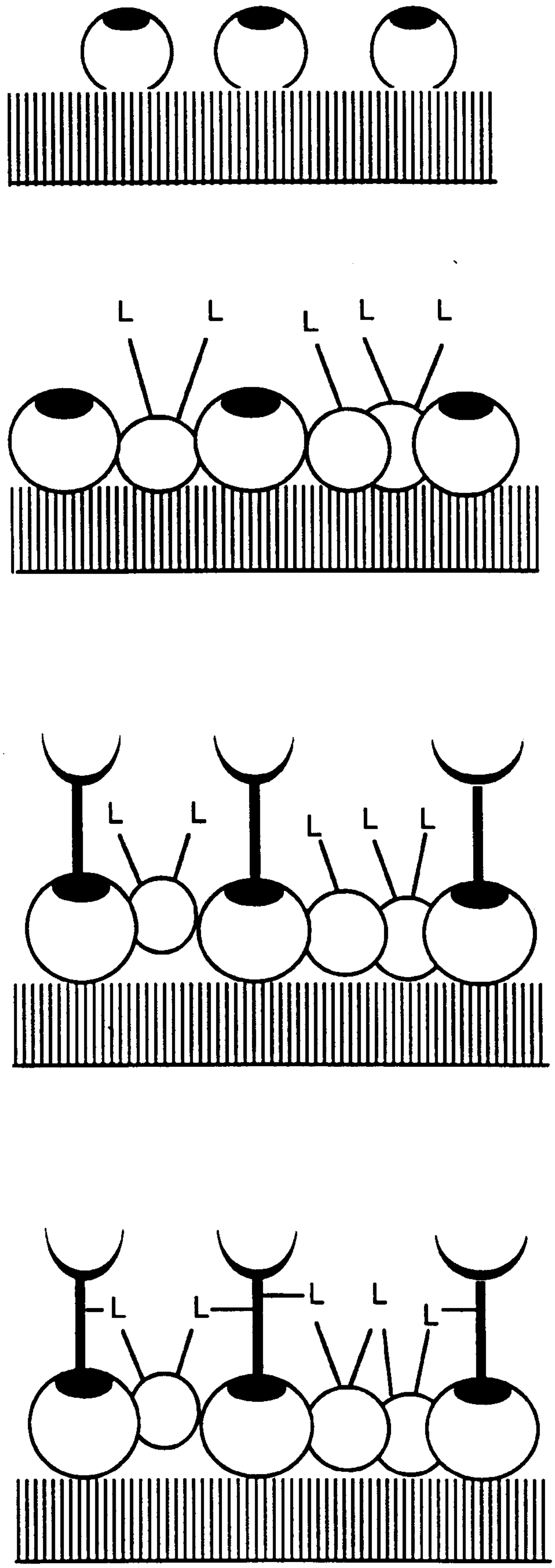


a)

b)

c)

d)

Fig. 2

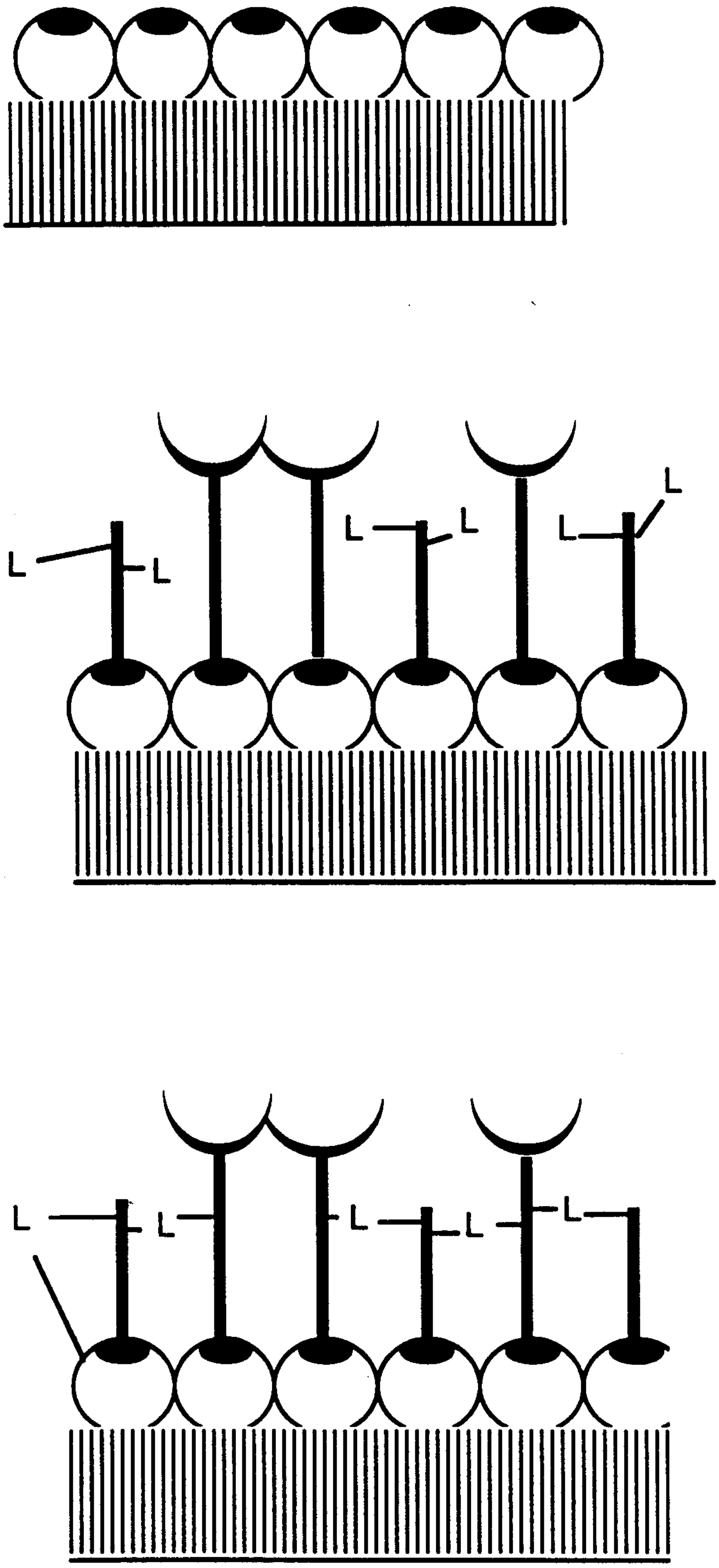


a)

b)

c)

**Fig. 3**